# FASCICULE DE BREVET EUROPEEN

(11) **EP 0 948 548 B1**
(45) Date de publication et mention de la délivrance du brevet: **14.11.2001**
(21) Numéro de dépôt: 97953977.2
(22) Date de dépôt: 30.12.1997
(51) Int. Cl.: C08F 8/44, C08F 20/34, C08F 20/60

(54) **POLYMERES ANTIMICROBIENS COMPORTANT DES GROUPES AMMONIUM QUATERNAIRE, LEUR UTILISATION POUR LA FABRICATION D'UN MATERIAU A PROPRIETES ANTIMICROBIENNES ET LEURS PROCEDES DE PREPARATION**
QUARTENÄRE AMMONIUMGRUPPEN ENTHALTENDE ANTIBAKTERIELLE POLYMERE,VERWENDUNG DAVON ZUR HERSTELLUNG VON ANTIBAKTERIELLEM MATERIAL
ANTIMICROBIAL POLYMERS COMPRISING QUATERNARY AMMONIUM GROUPS, THEIR USE FOR MAKING A MATERIAL WITH ANTIMICROBIAL PROPERTIES AND METHODS FOR PREPARING THEM

(30) Priorité: 30.12.1996 FR 9616362
(43) Date de publication de la demande: 13.10.1999
(73) Titulaire: Catalyse, 13013 Marseille (FR)
(72) Inventeur: PERICHAUD, Alain, F-13013 Marseille (FR); BATAILLE, Florence, F-13008 Marseille (FR); BAUDRION, Christophe, F-13008 Marseille (FR); PANAIVA, Lionel, F-13090 Aix en Provence (FR)
(74) Mandataire: Domange, Maxime
(86) Numéro de dépôt international: FR9702459
(87) Numéro de publication internationale: WO9829463

(56) Documents cités:
- EP-A- 0 187 281
- EP-A- 0 373 494
- EP-A- 0 385 627
- EP-A- 0 415 055
- EP-A- 0 525 751
- EP-A- 0 610 955
- WO-A-91/18027
- WO-A-94/27620
- DE-A- 4 022 651
- DE-A- 4 242 082
- GB-A- 2 010 851

## Description

L'invention concerne des polymères non réticulés antimicrobiens comportant des groupements ammonium quaternaire liés par une liaison potentiellement réactive avec l'eau, ayant une activité antimicrobienne, notamment antibactérienne.

L'invention concerne également leurs procédés de préparation ainsi que leurs applications pour l'obtention de matériaux antibactériens, ayant des propriétés auto-lissantes, auto-glissantes ou anti-statiques ou à titre de liants pour revêtements ou peintures.

L'obtention de polymères comportant des groupes ammonium quaternaire est décrite dans un certain nombre de brevets ou demandes de brevet.

WO 95/27473 décrit un polymère à squelette carboné, comportant des atomes d'azote quaternisés, dont un des substituants est hydrophobe et a au moins huit atomes de carbon), ayant une activité antitranspiration.

DE 4 242 082 décrit des copolymères d'acrylamide ou méthacrylamide comportant des groupes ammonium quaternaire dont le taux ne doit pas dépasser 20% de la masse du produit final.

EP 0 611 782 décrit un homo- ou copolymère contenant des groupes ammonium quaternaire antimicrobiens, mais comprenant entre la chaîne polymérique et le groupement actif une ou plusieurs liaisons silicium-carbone ou silicium-oxygène qui donnent au produit final des propriétés particulières.

WO 91/09915 décrit une composition antisalissure constituée d'un liant hydrolysable, contenant des groupements acides sulfoniques et sels d'ammonium quaternaire (ces deux derniers étant liés par une liaison ionique et non covalente).

EP 0 270 465 concerne des ammonium quaternaire greffés sur une résine vinylique chlorée par une liaison non hydrolysable ou non sensible à l'eau.

EP 0 156 632 décrit un copolymère comprenant des esters acryliques ou méthacryliques qui peuvent éventuellement renfermer des fonctions ammonium quaternaire, et contenant des composés organostanniques introduits par copolymérisation , dans une application de peintures sous-marines anti-salissures.

WO 84/02915 décrit des polymères comprenant des esters acryliques ou méthacryliques comprenant des motifs hydrolysables et pouvant éventuellement renfermer des fonctions ammonium quaternaire, dans une application de peintures sous-marines anti-salissures.

D'autres publications, notamment EP 0 494 554, EP 0 657 478, et EP 0 373 852, concernent des ammoniums quaternaires (majoritairement à chaîne courte inférieure à 4 carbones) liés à un squelette acrylique. Toutefois, ces documents décrivent des produits réticulés et parfois copolymérisés avec d'autres monomères vinyliques. Dans tous les cas le taux d'ammonium quaternaire est faible.

On a maintenant trouvé que des polymères comportant des ammoniums quaternaires en quantité prépondérante présentent une activité anti-microorganismes, en particulier antibactérienne, optimale. De plus, du fait du caractère potentiellement hydrolysable de la liaison (de type amide ou ester) des groupements ammoniums quaternaires avec la résine, ils sont susceptibles de conférer aux matériaux ou revêtements qui les contiennent un caractère auto-régénérant.

L'invention concerne donc selon un premier aspect des polymères non réticulés caractérisés en ce qu'ils sont constitués d'une résine ester et/ou amide à laquelle sont liés par une liaison covalente potentiellement réactive avec l'eau des sels d'ammonium quaternaire et dans lesquels le taux d'ammonium quaternaire est d'au moins 1 mole/kg, de préférence au moins 2 moles/kg et en particulier d'au moins 3 moles/kg, pour leur utilisation en tant qu'agents antimicrobiens, notamment antibactériens.

Dans un aspect préféré, le pourcentage pondéral de sels d'ammonium quaternaire est d'au moins 80 % et avantageusement jusqu'à 100 % de la masse du polymère.

L'invention concerne donc dans un aspect particulier des polymères non réticulés caractérisés en ce qu'ils sont constitués d'une résine ester et/ou amide à laquelle sont liés par une liaison covalente potentiellement réactive avec l'eau des sels d'ammonium quaternaire, de formule générale (I) : 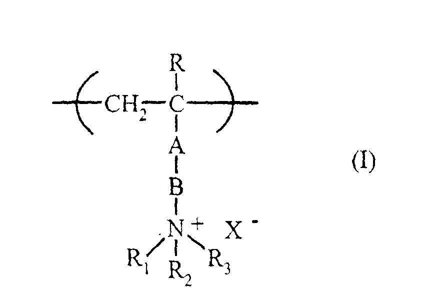 dans laquelle :
- A représente une fonction 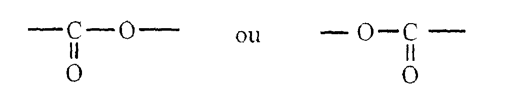 et/ou une fonction amide 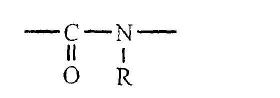
- R représente H ou CH₃ ;
- B représente une. chaîne C₀-C₅ alkylène, linéaire ou ramifiée ou un groupe arylène ou arylalkylène ;
- R₁ et R₂, identiques ou différents, représentent chacun une chaîne C₁-C₅ alkyle;
- R₃ représente une chaîne C₈-C₂₀ alkyle ou un groupe aryle ou arylalkyle :
- X⁻ représente un anion,
dans lesquels le taux d'ammonium quaternaire est supérieur à 1 mole/kg.

Selon un autre aspect, l'invention concerne des polymères non réticulés caractérisés en ce qu'ils sont constitués d'une résine ester et/ou amide à laquelle sont liés par une liaison covalente potentiellement réactive avec l'eau des sels d'ammonium quaternaire. de formule (II) ci-dessous 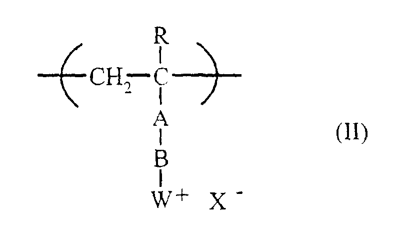 dans laquelle
- A représente une fonction 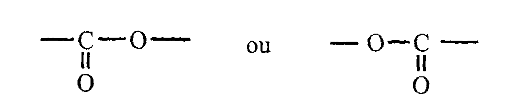 et/ou une fonction amide 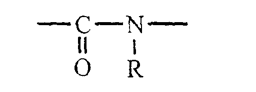
- R représente H ou CH₃ ;
- B représente une chaîne C₀-C₅ alkylène, linéaire ou ramifiée ou un groupe arylène ou arylalkylène ;
- W ⁺ est un hétérocycle saturé ou insaturé comprenant un atome d'azote substitué par R₄, ou directement lié à A ou à B, et pouvant également contenir en plus de l'azote quaternisé un ou plusieurs hétéroatomes, identiques ou différents,
- R₄ représente une chaîne C₁-C₂₀ alkyle ou un groupe aryle ou aralkyle ;
- X⁻ représente un anion ;
dans lesquels le taux d'ammonium quaternaire est supérieur à 1 mole/kg.

De préférence , le nombre total d'atomes de l'hétérocycle constitutif de W⁺ est de 3 à 15.

Avantageusement, W⁺ comprend un hétérocycle choisi parmi la pipéridine, la pipérazine, la morpholine, la thiomorpholine ou encore le thiazole, l'isothiazole, le pyrazole, l'indole, l'indazole, l'imidazole, le benzimidazole, la quinoléine. l'isoquinoléine, le benzotriazole, le benzothiazole, le benzoisothiazole, le benzoxazole, la benzoxazine, l'isoxazole, le pyrrole, la pyrazine, la pyrimidine, la pyridazine, la quinazoline, l'acridine, dont une ou plusieurs doubles liaisons peuvent être hydrogénées, lesdits groupes pouvant être non substitués ou substitués une ou plusieurs fois, par exemple, non limtativement, par un substituant choisi parmi les groupes alkyle, halogène, cyano, nitro, hydroxy, sulfone, trifluorométhyl.

Lorsque B représente un groupe arylène ou arylalkylène et/ou R₃ est un groupe aryle ou arylalkyle, le cycle aromatique peut par exemple être un groupe phényle et la chaîne alkyle peut être en C₁-C₅.

Selon un aspect avantageux, l'invention concerne des polymères non réticulés caractérisés en ce qu'ils sont constitués d'une résine ester et/ou amide à laquelle sont liés par une liaison covalente potenticllement réactive avec l'eau des sels d'ammonium quaternaire, comportant à la fois des motifs de formule (I) et (II) telles que définies plus haut.

Avantageusement, les polymères de formules (I) et/ou (II) ci-dessus comprennent un pourcentage pondéral de sels d'ammonium quaternaire d'au moins 80 %, avantageusement jusqu'à 100 % de la masse du polymère.

Ce pourcentage massique total comprend aussi bien les fonctions esters ou amides que le mélange des deux types de fonctions.

Dans un aspect préféré, le taux d'ammonium quaternaire est supérieur ou égal à 2 moles/kg et notamment supérieur ou égal à 3 moles/kg.

L'anion X⁻ est choisi parmi les anions habituellement utilisés dans le domaine et bien connus de l'homme du métier, tel que par exemple, de manière non limitative, halogène, sulfate, phosphate, nitrate, cyano, tosylate ou des anions métalliques ou organiques anioniques, tels que par exemple salicylate, benzoate, alcoolate, acétate, undécylénate.

L'invention concerne également les polymères de formules (I) et/ou (II) tels que définis plus haut, pour leur utilisation en tant qu'agents antimicrobiens, notamment antibactériens.

Selon un aspect ultérieur, l'invention concerne également l'utilisation des polymères décrits ci-dessus, en particulier des polymères de formules (I) et/ou (II) pour réaliser des objets proprement dits avec lesdits polymères, en particulier dans le domaine médical pour la fabrication de matériels médicaux tels que par exemple des cathéters, des sondes gastriques, des poches de recueil de sang ou même des dalles de sol, des semelles, des joints de carrelage, ou tout objet ou matériau dont il est souhaitable qu'il possède des propriétés antimicrobiennes, notamment antibactériennes.

L'invention concerne également l'utilisation des polymères décrits ci-dessus, en particulier des polymères de formules (I) et/ou (II) pour la fabrication de peintures, non seulement marines, mais également pour des salles blanches par exemple, ou encore des revêtements de façades, soit en fait partout où il y a des risques de développement de microorganismes et en particulier de bactéries contre lesquels on veut se prémunir.

Plus particulièrement, l'invention concerne des peintures ou des revêtements comprenant à titre de liant au moins un polymère décrit ci-dessus, en particulier au moins un polymère de formules (I) et/ou (II), en association avec un support volatile. Pour cette application, les polymères selon l'invention sont utilisés en association avec les composants habituels des peintures tels que notamment des solvants, par exemple un étheralcool, le xylène ou le cyclohexane, et des pigments tels que par exemple le sultate de baryum, l'oxyde de titane ou de fer.

La liaison potentiellement réactive avec l'eau, c'est à dire hydrolysable, entre les groupes ammonium quaternaires et la résine des polymères selon l'invention présente un intérêt supplémentaire dans la mesure où elle permet une régénération couche par couche de tout matériau à base du polymère de formules (I) et/ou (II), soit qu'il se trouve sous forme d'un revêtement d'un support ou soit en tant qu'élément constitutif dudit matériau.

L'invention concerne donc également l'utilisation des polymères de formules (I) et/ou (II) pour la fabrication de matériaux auto-régénérants.

L'invention concerne également les procédés de préparation des polymères décrits ci-dessus.

On distingue 2 modes principaux de préparation :
- voie 1) la modification d'un polymère ou d'un copolymère par réaction avec un halogénure d'alkyle ;
- voie 2) la polymérisation ou la copolymérisation d'un monomère comportant un groupement ammonium quaternaire.

Les monomères utilisables pour obtenir les polymères de formule (I) dans l'une ou l'autre des voies de préparation, soit en vue de leur polymérisation ou copolymérisation avant quatemisation, soit en vue de leur quaternisation préalable, sont de préférence choisis parmi les acrylates, méthacrylates, éthylméhacrylate, éthylméthacrylates, butylméthacrylates, 2-ethylhexylméthacrylates, méthoxyéthylméthacrylates, acétates, acrylamides, méthacrylamides, anhydrides maléique et alcool vinylique.

Pour obtenir les polymères de formule(II), on utilisera des monomères de type méthacryloyl- ou acryloylpyrazole connus dans le domaine, tels que décrits par exemple dans Macromol. Chem., 186, 1985, 1605-1611.

Selon une première variante de la voie 1 (voie 1a), la modification chimique s'effectue en une étape sur un polymère de type polyacrylate comportant une fonction amine tertiaire tel que par exemple le polyméthacrylate de 2-diméthylaminoéthyle ou le poly 3-diméthylaminopropylméthacrylamide. Ces précurseurs sont obtenus par des modes de polymérisation connus dans le domaine. Après dissolution de ces polymères dans des solvants de type polaire, tel que par exemple le méthanol, le propylène glycolmonométhyléther ou un mélange eau/acétone, on peut quaterniser les fonctions amines tertiaires par un halogénure d'alkyle selon le schéma 1a ci-dessous, dans lequel A, B, R₁, R₂ et R₃ sont tels que définis dans la formule (I) et X représente un atome d'halogène :

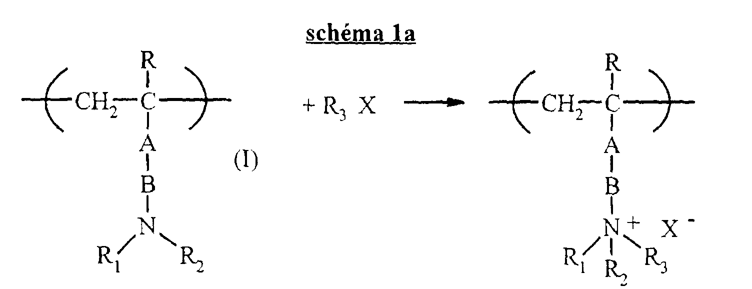

L'halogénure d'alkyle représenté par R₃X comporte une longue chaîne carbonée linéaire ou substituée par un cycle aromatique, tel que par exemple le bromure d'octyle, de lauryle ou de benzyle.

La voie la est également utilisable pour préparer les polymères de formule (II), en partant d'un polymère de type polyacrylate comportant une fonction amine tertiaire de formule 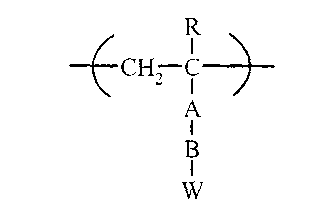 dans laquelle W représente un hétérocycle saturé ou insaturé comprenant un atome d'azote et pouvant également contenir en plus de l'azote un ou plusieurs hétéroatomes, identiques ou différents.

Dans ce cas, l'halogénure d'alkyle est représenté par la formule R₄X, dans laquelle R₄ est tel que défini plus haut pour la formule (II).

La concentration du polymère initial dépend de sa masse moléculaire en poids, qui est comprise entre environ 50 000 et environ 100 000, et varie en général de 1 à 50% par rapport à l'ensemble de la solution, y compris le solvant. La concentration en halogénure d'alkyle est fonction du taux d'ammonium désiré et varie de 1 à 100% par rapport au nombre de mole d'amine tertiaire quaternisable. Les rendements de réactions sont de l'ordre de 100% (calculés par des dosages coulométriques).

Selon une 2ème variante de la voie 1 (voie 1b), la transformation chimique est également réalisable à partir d'un polymère de type polyméthylméthacrylate (PMMA) par une trans-estérification avec une alcanolamine préalablement quaternisée. Selon le schéma réactionnel 1b ci-dessus dans lequel B, R, R₁, R₂, R₃ et X sont tels que définis dans la formule (I). Le polymère est solubilisé dans un solvant cétonique tel que par exemple la méthyléthylcétone, la méthylisoamylcétone ou la méthylisobutylcétone à une concentration variant de 1 à 50%. On ajoute à froid l'éthanolamine quaternisée avec un catalyseur tel que par exemple de l'oxyde de dioctyle étain.

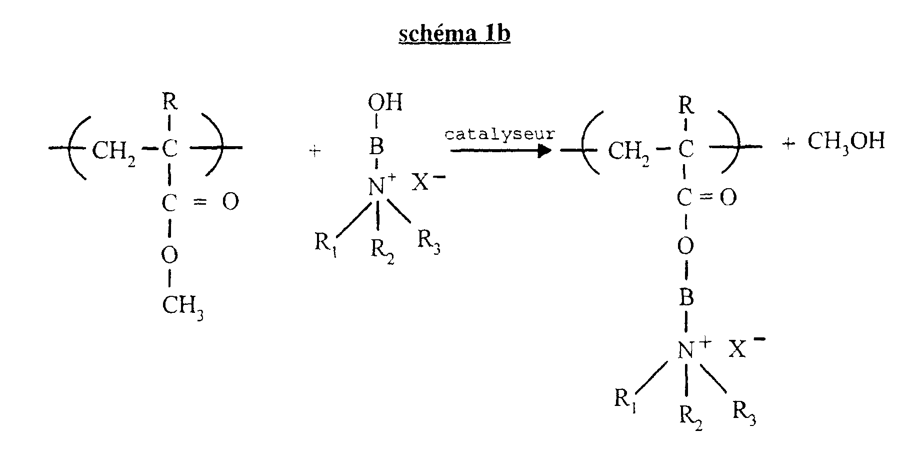

La solution est chauffée pendant 10 h à 100°C et le méthanol formé est éliminé par un appareillage de type Dean Stark. En fin de manipulation on recueille un produit dont le taux d'ammonium (par motif) est proche de 100% suivant les rapports initiaux.

Selon une troisième variante de la voie 1 (voie 1c), la transformation chimique peut être effectuée par estérification sur l'acide polyacrylique. Ce polymère est dissout dans l'alcanolamine déjà quaternisée auquel on ajoute un catalyseur de réaction tel que de l'acide sulfurique et un solvant tel que du toluène ou du cyclohexane qui permettra d'éliminer l'eau formée.

Cette variante est représentée par le schéma réactionnel 1c ci-dessous, dans lequel B, R₁, R₂, R₃ et X sont tels que définis dans la formule (I) et R = H.

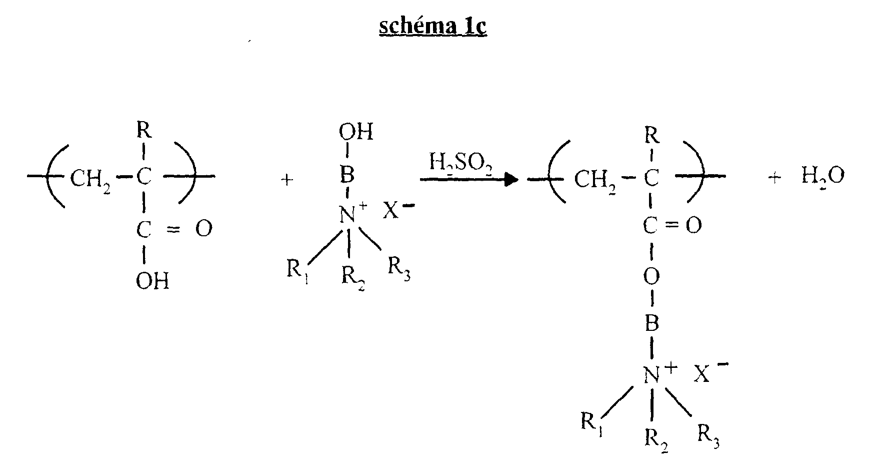

Après 5h d'une distillation azéotropique à 130°C, on récupère un mélange très visqueux (dont la masse moléculaire en poids est supérieure à environ 100 000) auquel on ajoute du tétrahydrofuranne. Le polymère entièrement quaternisé précipite.

Une quatrième variante de la voie 1, permettant d'obtenir des composés de formule (I) dans laquelle B, R₁, R₂ et R₃ sont tels que définis plus haut dans la formule (I) et R = H, consiste à faire réagir un polyalcool tel que le polyvinylalcool avec un anhydride d'acide halogéné, notamment chloré, puis à faire réagir dans une 2ème étape la fonction ester portant l'atome d'halogène avec une amine tertiaire pour obtenir l'ammonium quaternaire.

Comme indiqué plus haut, le deuxième mode d'obtention de polymères de formule (I) selon l'invention comportant des sels d'ammonium quaternaire est la polymérisation de monomères comportant des ammonium quaternaire, soit en phase solvant organique (voie 2a), soit en phase aqueuse (voie 2b).

Les monomères comportant des ammonium quaternaire sont dans un premier temps préparés en mélangeant en quantité équimoléculaire une amine de type méthacrylique ou méthacrylamide avec un halogénure d'alkyle tel que défini plus haut. La température de réaction est maintenue vers 100°C (la réaction est en phase homogène si un solvant organique est utilisé alors qu'initialement elle est hétérogène si c'est de l'eau). Le produit pur est récupéré par évaporation du solvant et recristallisation à chaud dans du tétrahydrofuranne.

La polymérisation des monomères comportant des ammonium quaternaire peut être effectuée en phase solvant organique par émulsion ou solution (voie 2a) : dans ce cas, on ajoute au produit obtenu de l'azobisisobutyronitrile (AIBN) et on chauffe à une température comprise entre environ 20°C et 200°C, de préférence environ 80°C .

Après 4 h de réaction, la viscosité du mélange a très fortement augmentée et la solution est refroidie à température ambiante. Le polymère peut être utilisé tel quel dans une formulation de peinture.

Lorsqu'on effectue la polymérisation en phase aqueuse en solution ou émulsion (voie 2b), les monomères comportant des ammonium quaternaire, solubles dans l'eau, sont polymérisés soit par addition de peroxyde, tel que par exemple AIBN ou peroxyde de benzoyle, soit à l'aide d'un amorçeur de type redox selon des techniques connues dans le domaine, en chauffant à une température comprise entre environ 20°C et 120°C. Des polymères ayant des masses moléculaires en poids élevées (≥ 100 000) peuvent être obtenus.

Le polymère formé précipite et est ensuite refroidi et séché ou, si on souhaite récupérer le polymère en phase organique pour l'utiliser directement dans une formulation de peinture, l'eau peut être éliminée par l'addition d'un solvant azéotropique tel qu'un étheralcool, par exemple le monopropylèneglycol-monométhyléther.

Le schéma réactionnel 2 ci-dessous, dans lequel R, R₁, R₂, R₃, A, B et X sont tels que définis plus haut pour la formule (I), représente de manière générale le 2ème mode d'obtention des polymères de formule (I), à savoir quaternisation des monomères et polymérisation.

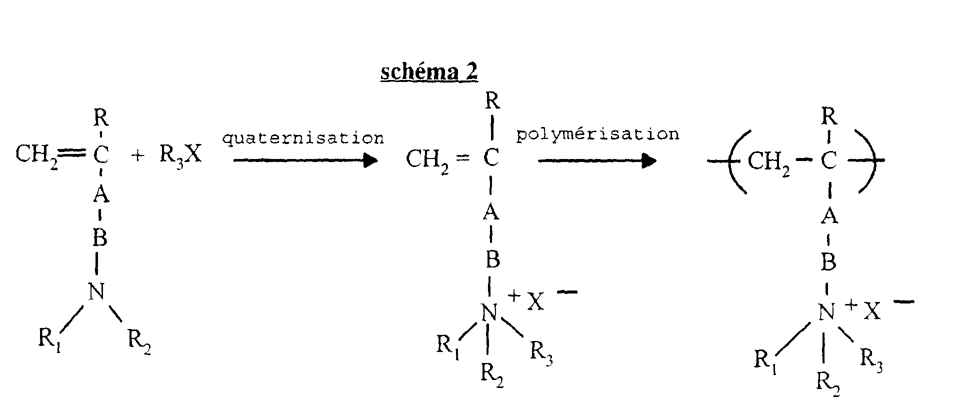

Ce schéma réactionnel est également applicable pour la préparation des polymères de formule (II), en partant de monomères de formule 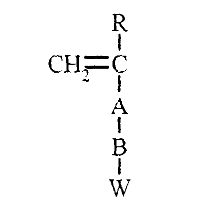 dans laquelle W est tel que défini plus haut.

L'invention est illustrée par les exemples ci-après.

### EXEMPLE 1

### Modification chimique d'un poly 2-diméthylaminoéthylméthacrylate (voie 1a)

Dans un ballon bicol équipé d'un réfrigérant et d'un agitateur magnétique, on introduit 18,86 g (soit 0,132 mole) de poly 2-diméthylaminoéthylméthacrylate. On additionne ensuite 12,7 g (0,066 mole) de bromooctane et 36 g d'acétone. Après 18 h. à 70°C, on ajoute 30 g de propylène glycol monométhyl éther et l'acétone est distillée. Le produit final se trouve alors avec un extrait sec de 51% prêt à l'emploi dans une formulation peinture marine. Un dosage coulométrique (ainsi qu'une analyse par spectrométrie infrarouge) indique la présence de 2,03 moles d'ammonium/kg de produit sec.

### EXEMPLE 2 :

### Estérification de l'acide polyacrylique (yoie 1c)

Dans un ballon bicol de 100 ml muni d'un distillateur azéotropique (type Dean-Stark) et d'un agitateur magnétique, on introduit à froid 20 g d'éthanolamine (au préalable quaternisée par du bromooctane) avec 5,1 g d'acide polyacrylique
(à 35% dans de l'eau et dont la masse moléculaire en nombre est de 1 000 g/mole). A ce mélange sont ajoutés 30 g de toluène et 1 ml d'acide sulfurique concentré. La solution est alors chauffée à 130°C pendant 3 h. où l'on recueille 4 cm³ d'eau.

Après refroidissement, le polymère est récupéré par précipitation dans de l'éther. La masse obtenue (9 g) est analysée par spectrométrie infrarouge qui nous indique la présence d'un carbonyle ester à 1730 cm⁻¹, soit 2,82 moles d'ammonium/kg de produit sec,

### EXEMPLE 3 :

### Modification du monomère puis polymérisation en phase organique (voie 2a)

Dans un réacteur de 3 litres, 200 g de 2-diméthylaminoéthyle méthacrylate sont ajoutés à 246 g de bromooctane dans 450 g de propylène glycol monométhyl éther. Après 3 h de réaction à 80°C, un dosage coulométrique permet de vérifier un taux de conversion de 100% en ion chlorure. Le monomère quaternisé est alors soit purifié par une précipitation dans l'hexane, soit utilisé pour une étape ultérieure (polymérisation).

A la solution issue de l'étape précédente, on ajoute 2 g d'azobis isobutyronitrile et la température est progressivement élevée. Après 1 h et 45 min. (soit à une température de 76°C), on note une augmentation de viscosité ; la réaction est alors maintenue à cette température pendant 3 h. Après refroidissement, on obtient un liant pour peinture dont l'extrait sec est de 50%. soit 2,75 mole d'ammonium/kg de produit sec.

### EXEMPLE 4 :

### Modification du monomère puis polymérisation en phase aqueuse (voie 2b)

Dans un réacteur de 3 litres, 400 g de 2-diméthylaminoéthyl méthacrylate sont ajoutés à 500 g de bromo octane et 200 g d'eau. Au départ, le milieu réactionnel qui est hétérogène est chauffé à 90°C et la réaction est suivie par dosage coulométrique. Après 4 h de réaction, le taux de conversion en ion chlorure est de 100% (la phase réactionnelle est limpide et homogène). Le produit est refroidi et utilisable pour la polymérisation.

A la solution aqueuse de l'étape précédente, on ajoute 400 g d'une solution aqueuse de gomme arabique à 1% et 5 g d'azo bis isobutyronitrile. Le mélange est porté à 80°C. Après 1 heure de réaction, la viscosité du mélange augmente très fortement et un précipité apparaît. Après 2 h à cette température, le réacteur est refroidi jusqu'à 30°C environ et le polymère qui se présente sous forme d'une pâte blanchâtre est séché sous vide. Après une semaine de séchage, on obtient un solide blanc dont l'analyse par spectrométrie infrarouge confirme la disparition des doubles liaisons du monomère initial et la présence de 2,75 moles d'ammonium/kg de produit sec. Le produit peut alors être dissout dans un solvant organique afin d'obtenir un liant pour peinture marine.

### EXEMPLE 5: Préparation d'un polymère de formule(II)

### 1) Préparation du monomère:

Dans un ballon tricol de 250 ml équipé d'une agitation et d'un réfrigérant, 5 g de chlorure de méthacryloyle (0,048 mole)sont mélangés à 100 ml de méthyl éthyl cétone (MEK). Sous agitation, mécanique, on ajoute goutte à goutte une solution de 4,8 g de 2-aminothiazole (0,048 mole) dans 50 ml de MEK. A la fin de l'addition, la solution est chauffée à 60°C pendant 4 h. Après refroidissement, 50 ml d'eau basique (hydrogénocarbonate de sodium) sont ajoutés de manière à neutraliser l'acide chlorhydrique formé. On récupère après évaporation du solvant 7,5 g de 2-thiazolylméthacrylamide.

### 2) Modification du monomère puis polymérisation (voie 2a)

Dans un bicol de 100 ml équipé d'une agitation magnétique et d'un réfrigérant, on ajoute 5 g (0,030 mole) de 2-thiazolylméthacrylamide obtenu à l'étape 1) à 14 g de propylène glycol monométhyléther et 4,26 g (0,030 mole) d'iodométhane. Après 10h de réaction à 60°C, un dosage coulométrique indique un taux de conversion de 100% en ion iodure. Le monomère quaternisé est alors soit purifié par une précipitation dans l'hexane, soit utilisé pour l'étape de polymérisation.

A la solution obtenue ci-dessus, on ajoute sous agitation mécanique 0,06 g d'azobisisobutyronitrile et la température est progressivement élevée à 85°C. Après 4 h à cette température, on note une augmentation de la viscosité du mélange. Après refroidissement de la solution, on obtient un liant avec un extrait sec de 40 % prêt à l'emploi pour une fabrication de peinture. Le polymère peut aussi être précipité dans le pentane de manière à obtenir un produit ayant un taux d'ammonium de 3,22 moles/kg sec.

### EXEMPLE 6 :

### Etude des propriétés antibactériennes des polymères selon l'invention

Le principe de dosage est la mise en contact de la résine qui est un homopolymère à 100% d'ammoniums quaternaires par motif constitutif (sur une surface fixe) avec un innoculum connu d'une souche bactérienne ou fongique (Staphylococcus aureus, Streptococcus faecalis, Escherichia coli, Pseudomonas aeruginosa ou Candida albicans) pendant un temps de contact t (1 ou 30 min).

Le liant est coulé dans des puits stériles puis le solvant est chassé sous vide à chaud pendant une semaine. Un nombre connu de colonies bactériennes est alors mis en contact, puis une partie est prélevée et mise en incubation pendant 48 heures. A ce stade on dénombre alors les colonies restantes.
Polymère 1 : Bromure de N-octyl N,N-diméthylaminopropylméthacrylamide
Polymère 2 : Bromure de N-dodecyl N,N-diméthylaminopropyl méthacrylamide
Polymère 3 : Bromure de N-hexadecyl N,N-diméthylaminopropylméthacrylamide
Polymère 4 : Chlorure de N-octyl N,N-diméthylaminopropylméthacrylamide
Polymère 5 : Chlorure de N-dodécyl N,N-diméthylaminopropylméthacrylamide
Polymère 6 Chlorure de N-hexadécyl N,N-diméthylaminopropylméthacrylamide
Copolymère 7 : co bromure de N-octyl N-N-diméthyl aminoéthylméthacrylate (47%) co méthacrylate (53%)
Copolymère 8 : co bromure de N-octyl N,N-diméthyl aminoéthylméthacrylate (65%) co méthacrylate (45%)

Les résultats rapportés dans le tableau 1 ci-dessous sont ceux d'un dosage réalisé avec un innoculum de Staphylococcus aureus, le nombre initial de colonies étant N₀ = 55.10⁶.

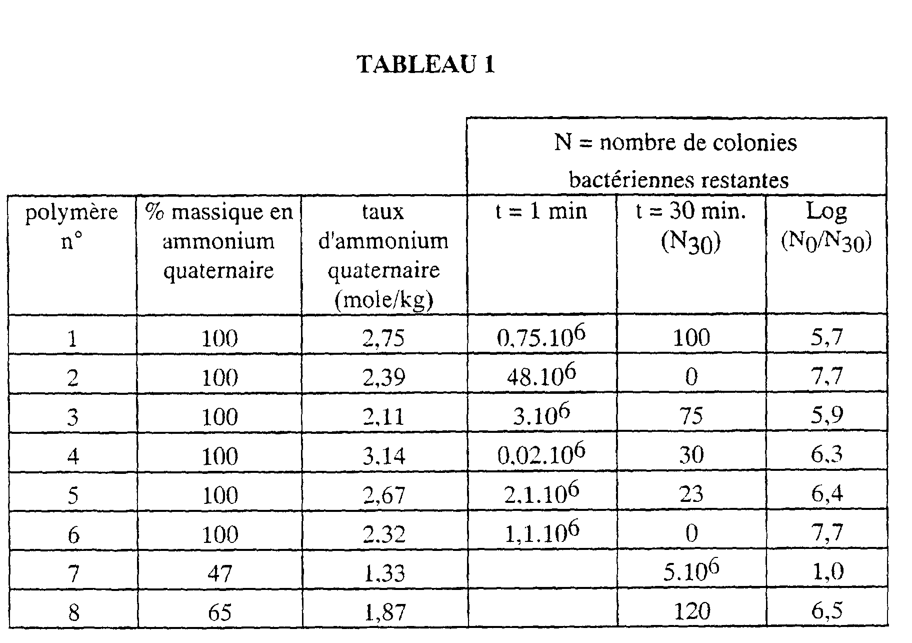

Les résultats montrent que les polymères dans lesquels le taux d'ammonium quaternaire est supérieur à 1 mole/kg présente une importance activité antibactérienne.

## Revendications

1. Utilisation de polymères non réticulés constitués d'une résine ester et/ou amide à laquelle sont liés par une liaison covalente potentiellement réactive avec l'eau des sels d'ammonium quaternaire, le taux d'ammonium quaternaire étant d'au moins 1 mole/kg, comme agents antimicrobiens.

2. Utilisation selon la revendication 1, **caractérisée en ce que** lesdits polymères sont constitués d'une résine ester et/ou amide à laquelle sont liés par une liaison covalente potentiellement réactive avec l'eau des sels d'ammonium quaternaire, de formule générale (I): 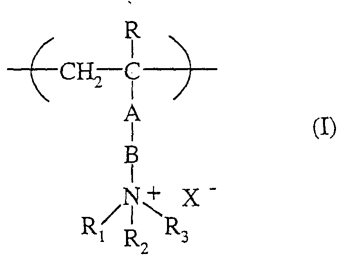 dans laquelle :
- A représente une fonction 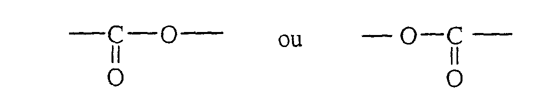 et/ou une fonction amide 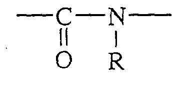
- R représente H ou CH₃ ;
- B représente une chaîne C₀-C₅ alkylène, linéaire ou ramifiée, ou un groupe arylène ou arylalkylène.
- R₁ et R₂, identiques ou différents, représentent chacun une chaîne C₁-C₅ alkyle;
- R₃ représente une chaîne C₈-C₂₀ alkyle ou un groupe aryle ou arylalkyle ;
- X⁻ représente un anion,
dans lesquels le taux d'ammonium quaternaire est d'au moins 1 mole/kg.

3. Utilisation selon les revendications 1 ou 2, **caractérisée en ce que** le taux d'ammonium quaternaire des polymères est d'au moins 2 moles/kg, en particulier d'au moins 3 moles/kg.

4. Utilisation selon la revendication 3, **caractérisée en ce que**, lorsque B représente un groupe arylène ou arylalkylène et/ou R₃ est un groupe aryle ou arylalkyle, le cycle aromatique est un groupe phényle et la chaîne alkyle est en C₁-C₅.

5. Utilisation selon l'une quelconque des revendications 1 à 4, **caractérisée en ce que** le pourcentage pondéral de sels d'ammonium quaternaire du polymère est d'au moins 80% de la masse du polymère.

6. Utilisation selon l'une quelconque des revendications 1 à 5, **caractérisée en ce que** le pourcentage pondéral de sels d'ammonium quaternaires est de 100 % de la masse du polymère.

7. Utilisation selon l'une quelconque des revendications 1 à 6 d'un polymère choisi parmi le bromure de N-octyl N,N-diméthylaminopropylméthacrylamide, le bromure de N-dodecyl N,N-diméthylaminopropylméthacrylamide, le bromure de N-hexadecyl N,N-diméthylaminopropylméthacrylamide, le chlorure de N-octyl N,N-diméthylaminopropylméthacrylamide, le chlorure de N-dodécyl N,N-diméthylaminopropylméthacrylamide, le chlorure de N-hexadécyl N,N-diméthylaminopropylméthacrylamide, le co bromure de N-octyl N-N-diméthylaminoéthylméthacrylate (47%) co méthacrylate (53%) et le co bromure de N-octyl N,N-diméthyl aminoéthylméthacrylate (65%) co méthacrylate (45%).

8. Polymères non réticulés **caractérisés en ce qu'**ils sont constitués d'une résine ester et/ou amide à laquelle sont liés par une liaison covalente potentiellement réactive avec l'eau des sels d'ammonium quaternaire, de formule générale (II) 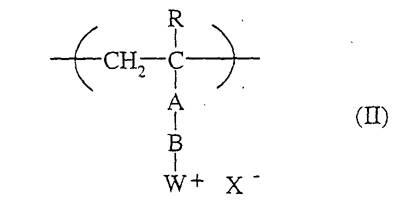 dans laquelle
- A représente une fonction 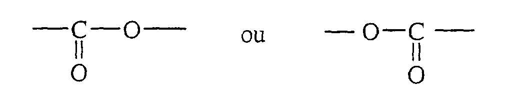 et/ou une fonction amide 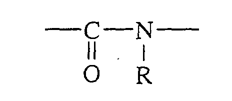
- R représente H ou CH₃ ;
- B représente une chaîne C₀-C₅ alkylène, linéaire ou ramifiée ou un groupe arylène ou arylalkylène ;
- W⁺ est un hétérocycle saturé ou insaturé comprenant un atome d'azote substitué par R₄, ou directement lié à A ou à B, et pouvant également contenir en plus de l'azote quaternisé un ou plusieurs hétéroatomes, identiques ou différents,
- R₄ représente une chaîne C₁-C₂₀ alkyle ou un groupe aryle ou aralkyle ;
- X⁻ représente un anion ;
dans lesquels le taux d'ammonium quaternaire est supérieur à 1 mole/kg.

9. Polymères **caractérisés en ce qu'**ils sont constitués d'une résine ester et/ou amide à laquelle sont liés par une liaison covalente potentiellement réactive avec l'eau des sels d'ammonium quaternaire, comportant à la fois des motifs de formule (I) et (II) telles que définies dans les revendications 2 et 8.

10. Polymères selon l'une quelconque des revendications 8 ou 9, **caractérisés en ce que** le taux d'ammonium quaternaire est d'au moins 2 moles/kg, notamment d'au moins 3 moles/kg.

11. Polymères selon la revendication 9, **caractérisés en ce que**, lorsque B représente un groupe arylène ou arylalkylène et/ou R₃ est un groupe aryle ou arylalkyle, le cycle aromatique est un groupe phényle et la chaîne alkyle est en C₁-C₅.

12. Polymères selon l'une quelconque des revendications 8 à 11, **caractérisés en ce que** le pourcentage pondéral de sels d'ammonium quaternaire du polymère est d'au moins 80% de la masse du polymère.

13. Polymères selon l'une quelconque des revendications 8 à 12, **caractérisés en ce que** le pourcentage pondéral de sels d'ammonium quaternaires est de 100 % de la masse du polymère.

14. Polymères selon l'une quelconque des revendications 8 à 13, pour leur utilisation en tant qu'agents antimicrobiens.

15. Utilisation des polymères tels que définis dans l'une quelconque des revendications 1 à 14, pour la fabrication d'objets ayant des propriétés antimicrobiennes.

16. Utilisation selon la revendication 15, pour la fabrication de matériel médical.

17. Utilisation des polymères tels que définis dans l'une quelconque des revendications 1 à 14, pour la fabrication de peintures ou de revêtements.

18. Peinture comprenant à titre de liant au moins un polymère tel que défini dans l'une quelconque des revendications 1 à 14.

19. Revêtement de façade comprenant à titre de liant au moins un polymère tel que défini dans l'une quelconque des revendications 1 à 14.

20. Utilisation des polymères tels que définis dans l'une quelconque des revendications 1 à 14 pour la fabrication de polymères auto-régénérants.

21. Procédé d'obtention des polymères de formule (II) selon la revendication 8, **caractérisé en ce qu'**il consiste, dans une première étape, à faire réagir un monomère comportant une amine tertiaire de formule : 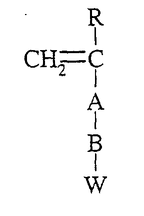 avec un halogénure d'alkyle de formule R₄X,
R, R₄, X, A, et B étant tels que définis dans la revendication 4, et W représente un hétérocycle saturé ou insaturé comprenant un atome d'azote et pouvant également contenir en plus de l'azote un ou plusieurs hétéroatomes, identiques ou différents,
et, dans une 2ème étape, à polymériser le monomère comportant un groupe ammonium quaternaire.

## Patentansprüche

1. Verwendung von nicht-vernetzten Polymeren, gebildet aus einem Harzester und/oder -amid an den bzw. das durch eine kovalente, potentiell mit Wasser reaktive Bindung, quaternäre Ammoniumsalze gebunden sind, wobei der Gehalt an quaternärem Ammonium wenigstens 1 Mol/kg beträgt, als antimikrobielle Mittel.

2. Verwendung nach Anspruch 1, **dadurch gekennzeichnet, dass** die genannten Polymere aus einem Esterharz und/oder -amid gebildet sind, an den bzw. das durch eine kovalente, potentiell mit Wasser reaktive Bindung quaternäre Ammoniumsalze der allgemeinen Formel (I) gebunden sind: 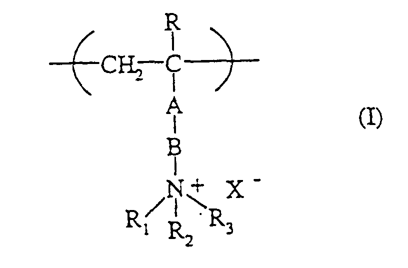 worin:
- A eine Funktion 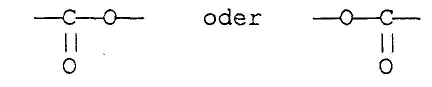 und/oder eine Amidfunktion 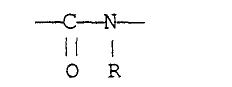 darstellt,
- R H oder CH₃ darstellt;
- B eine lineare oder verzweigte C₀-C₅-Alkylenkette darstellt oder eine Arylen- oder Arylalkylengruppe ist;
- R₁ und R₂, identisch oder verschieden voneinander, jeweils eine C₁-C₅-Alkylkette darstellen;
- R₃ eine C₈-C₂₀-Alkylkette oder eine Aryl- oder Arylalkylgruppe darstellt;
- X⁻ ein Anion darstellt,
worin der Gehalt an quaternärem Ammonium wenigstens 1 Mol/kg beträgt.

3. Verwendung nach den Ansprüchen 1 oder 2, **dadurch gekennzeichnet, dass** der Gehalt an quaternärem Ammonium der Polymere wenigstens 2 Mol/kg, insbesondere wenigstens 3 Mol/kg, beträgt.

4. Verwendung nach Anspruch 3, **dadurch gekennzeichnet, dass**, wenn B eine Arylen- oder Arylalkylengruppe darstellt und/oder R₃ eine Aryl- oder Arylalkylgruppe ist, der aromatische Ring eine Phenylgruppe ist und die Alkylkette 1 bis 5 Kohlenstoffatome aufweist.

5. Verwendung nach einem der Ansprüche 1 bis 4, **dadurch gekennzeichnet, dass** der Gewichtsprozentsatz der quaternären Ammoniumsalze des Polymeres wenigstens 80 % der Polymermasse ausmacht.

6. Verwendung nach einem der Ansprüche 1 bis 5, **dadurch gekennzeichnet, dass** der Gewichtsprozentsatz der quaternären Ammoniumsalze 100 % der Polymermasse ausmacht.

7. Verwendung nach einem der Ansprüche 1 bis 6 eines Polymeres, ausgewählt unter dem Bromid von N-Octyl-N,N-dimethylaminopropylmethacrylamid, dem Bromid von N-Dodecyl-N,N-dimethylaminopropylmethacrylamid, dem Bromid von N-Hexadecyl-N,N-dimethylaminopropylmethacrylamid, dem Chlorid von N-Octyl-N,N-dimethylaminopropylmethacrylamid, dem Chlorid von N-Dodecyl-N,N-dimethylaminopropylmethacrylamid, dem Chlorid von N-Hexadecyl-N,N-dimethylaminopropylmethacrylamid, dem Co-Bromid von N-Octyl-N,N-dimethylaminoethylmethacrylat (47 %)-Co-Methacrylat (53 %) und dem Co-Bromid von N-Octyl-N,N-dimethylaminoethylmethacrylat (65 %)-Co-Methacrylat (45 %).

8. Nicht-vernetzte Polymere, **dadurch gekennzeichnet, dass** sie aus einem Harzester und/oder -amid gebildet sind, an den bzw. das durch eine kovalente, potentiell mit Wasser reaktive Bindung quaternäre Ammoniumsalze der allgemeinen Formel (II) gebunden sind: 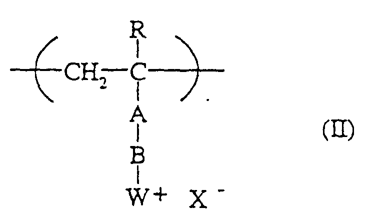 worin
- A eine Funktion 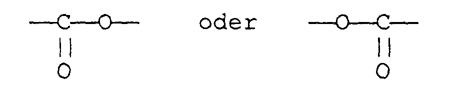 und/oder eine Amidfunktion 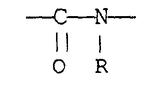 darstellt,
- R H oder CH₃ darstellt;
- B eine lineare oder verzweigte C₀-C₅-Alkylenkette oder eine Arylen- oder Arylalkylengruppe darstellt;
- W⁺ ein gesättigter oder ungesättigter Heterozyklus ist, umfassend ein durch R₄ substituiertes Stickstoffatom, oder direkt mit A oder B verbunden und der auch neben dem quaternisierten Stickstoff eines oder mehrere Heteroatome, welche identisch oder verschieden voneinander sind, enthalten kann;
- R₄ eine C₁-C₂₀-Alkylkette oder eine Aryl- oder Arylalkylgruppe darstellt;
- X⁻ ein Anion darstellt,
worin der Gehalt an quaternärem Ammonium über 1 Mol/kg beträgt.

9. Polymere, **dadurch gekennzeichnet, dass** sie aus einem Harzester und/oder -amid gebildet sind, an den bzw. das durch eine kovalente, potentiell mit Wasser reaktive Bindung quaternäre Ammoniumsalze gebunden sind, gleichzeitig umfassend Bausteine der Formel (I) und (II), wie sie in den Ansprüchen 2 und 8 definiert sind.

10. Polymere nach einem der Ansprüche 8 oder 9, **dadurch gekennzeichnet, dass** der Gehalt an quaternärem Ammonium wenigstens 2 Mol/kg, insbesondere wenigstens 3 Mol/kg, beträgt.

11. Polymere nach Anspruch 9, **dadurch gekennzeichnet, dass** wenn B eine Arylen- oder Arylalkylengruppe darstellt und/oder R₃ eine Aryl- oder Arylalkylgruppe ist, der aromatische Ring eine Phenylgruppe ist und die Alkylkette 1 bis 5 Kohlenstoffatome aufweist.

12. Polymere nach einem der Ansprüche 8 bis 11, **dadurch gekennzeichnet, dass** der Gewichtsprozentsatz von quaternärem Ammonium des Polymeres wenigstens 80 % der Polymermasse ausmacht.

13. Polymere nach einem der Ansprüche 8 bis 12, **dadurch gekennzeichnet, dass** der Gewichtsprozentsatz der quaternären Ammoniumsalze 100 % der Polymermasse ausmacht.

14. Polymere nach einem der Ansprüche 8 bis 13 zur Verwendung als antimikrobielles Mittel.

15. Verwendung der Polymere wie in einem der Ansprüche 1 bis 14 definiert, zur Herstellung von Gegenständen mit antimikrobiellen Eigenschaften.

16. Verwendung nach Anspruch 15 zur Herstellung von medizinischem Material.

17. Verwendung der Polymere, wie sie in einem der Ansprüche 1 bis 14 definiert sind, zur Herstellung von Anstrichfarben oder Aus- bzw. Verkleidungen.

18. Anstrichfarbe, umfassend als Bindemittel ein Polymer wie es in einem der Ansprüche 1 bis 14 definiert ist.

19. Aus- bzw. Verkleidung von Fassaden, umfassend als Bindemittel wenigstens ein Polymer wie es in einem der Ansprüche 1 bis 14 definiert ist.

20. Verwendung von Polymeren wie in einem der Ansprüche 1 bis 14 definiert, zur Herstellung von Auto-Regenerierungsmitteln.

21. Verfahren zum Erhalten von Polymeren der Formel (II) nach Anspruch 8, **dadurch gekennzeichnet, dass** es in einem ersten Schritt darin besteht, ein Monomer, umfassend ein tertiäres Amin der Formel: 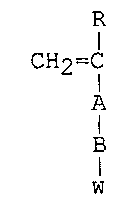 mit einem Alkylhalogenid der Formel R₄X umzusetzen,
wobei R, R₄, X, A und B die im Anspruch 4 angegebene Bedeutung besitzen und W ein gesättigter oder ungesättigter Heterozyklus ist, der ein Stickstoffatom umfasst und auch neben dem Stickstoff ein oder mehrere Heteroatome, welche identisch oder verschieden voneinander sind, enthalten kann,
und in einem zweiten Schritt darin, das eine quaternäre Ammoniumgruppe enthaltende Monomer zu polymerisieren.

## Claims

1. Use, as antimicrobial agents, of non-cross-linked polymers which are constituted of an ester and/or amide resin to which quaternary ammonium salts are bound by a covalent bond which is potentially reactive with water, the quaternary ammonium content being at least 1 mole/kg.

2. Use according to claim 1, **characterised in that** said polymers are constituted of an ester and/or amide resin, to which quaternary ammonium salts are bound by a covalent bond which is potentially reactive with water, of general formula (I): 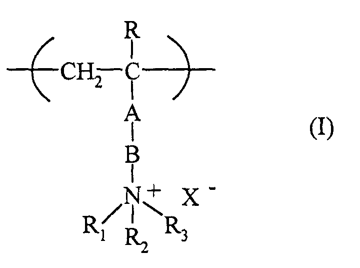 in which :
- A represents a 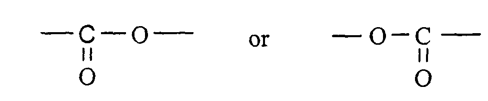 function,
and/or an amide function : 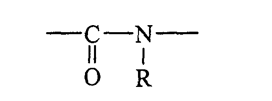
- R represents H or CH₃ ;
- B represents a C₀-C₅ alkylene chain, which is linear or branched, or an arylene or arylalkylene group;
- R₁ and R₂, which are identical or different, each represent a C₁-C₅ alkyl chain ;
- R₃ represents a C₈-C₂₀ alkyl chain or an aryl or arylalkyl group ;
- X⁻ represents an anion ;
in which polymers the quaternary ammonium content is at least 1 mole/kg.

3. Use according to claim 1 or 2, **characterised in that** the quaternary ammonium content of the polymers is at least 2 moles/kg, in particular at least 3 moles/kg.

4. Use according to claim 3, **characterised in that**, when B represents an arylene or arylalkylene group and/or R₃ is an aryl or arylalkyl group, the aromatic ring is a phenyl group and the alkyl chain is a C₁-C₅ alkyl chain.

5. Use according to any one of claims 1 to 4, **characterised in that** the weight percentage of quaternary ammonium salts is at least 80% of the mass of the polymer.

6. Use according to any one of claims 1 to 5, **characterised in that** the weight percentage of quaternary ammonium salts is 100 % of the mass of the polymer.

7. Use according to one of claims 1 to 6 of a polymer selected from N-octyl N,N-dimethylaminopropylmethacrylamide bromide, N-dodecyl N,N-dimethylaminopropyl methacrylamide bromide, N-hexadecyl N,N-dimethylaminopropylmethacrylamide bromide, N-octyl N,N-dimethylaminopropylmethacrylamide chloride, N-dodecyl N,N-dimethylaminopropylmethacrylamide chloride, N-hexadecyl N,N-dimethylaminopropylmethacrylamide chloride, N-octyl N-N-dimethyl aminoethylmethacrylate bromide (47%)-methacrylate (53%) copolymer, and N-octyl N,N-dimethyl aminoethylmethacrylate bromide (65%)-methacrylate (45%) copolymer.

8. Non-cross-linked polymers, **characterised in that** they are constituted of an ester and/or amide resin to which quaternary ammonium salts are bound by a covalent bond which is potentially reactive with water, of general formula (II): 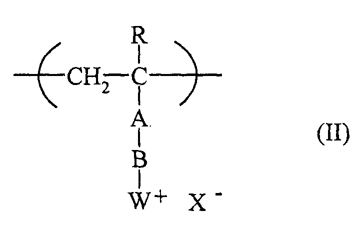 in which
- A represents a 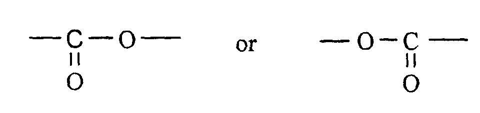 function, and/or an amide function : 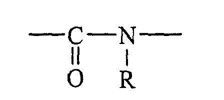
- R represents H or CH₃ ;
- B represents a C₀-C₅ alkylene chain, which is linear or branched or an arylene or arylalkylene group ;
- W⁺ is a saturated or unsaturated heterocycle comprising a nitrogen atom substituted with R₄, or directly bound to A or to B, and also able to contain, in addition to the quarternised nitrogen, one or more heteroatoms, which are identical or different,
- R₄ represents a C₁-C₂₀ alkyl chain or an aryl or aralkyl group ;
- X⁻ represents an anion ;
in which polymers the quaternary ammonium content is more than 1 mole/kg.

9. Polymers, **characterised in that** they are constituted of an ester and/or amide resin to which quaternary ammonium salts are bound by a covalent bond which is potentially reactive with water, comprising both units of formula (I) and (II) as defined in claims 2 and 8.

10. Polymers according to any one of claims 8 or 9, **characterised in that** the quaternary ammonium content is at least 2 moles/kg, notably at least 3 moles/kg.

11. Polymers according to claim 9, **characterised in that**, when B represents an arylene or arylalkylene group and/or R₃ is an aryl or arylalkyl group, the aromatic ring is a phenyl group and the alkyl chain is a C₁-C₅ alkyl chain.

12. Polymers according to any one of claims 8 to 11, **characterised in that** the weight percentage of quaternary ammonium salts is 80% of the mass of the polymer.

13. Polymers according to any one of claims 8 to 12, **characterised in that** the weight percentage of quaternary ammonium salts is 100% of the mass of the polymer.

14. Polymers according to any one of claims 8 to 13, for the use thereof as antimicrobial agents.

15. Use of the polymers as defined in any one of claims 1 to 14, for the preparation of objects having antimicrobial properties.

16. Use according to claim 15, for the preparation of medical material.

17. Use of the polymers as defined in any one of claims 1 to 14, for the preparation of paints or coatings.

18. Paint comprising at least one polymer according to any one of claims 1 to 14, as binder.

19. Facade coating comprising at least one polymer according to any one of claims 1 to 14, as binder.

20. Use of the polymers according to any one of claims 1 to 14 for the preparation of self-regenerating polymers.

21. Method of obtaining polymers of formula (II) according to claim 8, **characterised in that** it consists, in a first step, in allowing a monomer comprising a tertiary amine of formula : 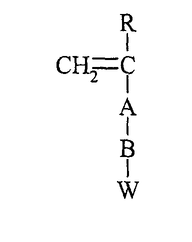 to react with an alkyl halide of formula R₄X,
R, R₄, X, A, and B being as defined in claim 4, and W represents a saturated or unsaturated heterocycle comprising a nitrogen atom and which may also contain, in addition to the nitrogen, one or more heteroatoms, which are identical or different,
and, in a 2nd step, in polymerising the monomer comprising a quaternary ammonium group.
